(19) Europäisches Patentamt
European Patent Office
Office européen des brevets

(11) EP 1 316 321 B1

(12) EUROPEAN PATENT SPECIFICATION

(45) Date of publication and mention of the grant of the patent:
**15.08.2007 Bulletin 2007/33**

(21) Application number: **00977962.0**

(22) Date of filing: **27.11.2000**

(51) Int Cl.:
***A61L 27/14*** (2006.01) ***A61L 27/18*** (2006.01)
***A61L 27/38*** (2006.01) ***A61L 27/52*** (2006.01)
***C12N 5/00*** (2006.01) ***C12N 5/06*** (2006.01)

(86) International application number:
**PCT/JP2000/008350**

(87) International publication number:
**WO 2002/002159 (10.01.2002 Gazette 2002/02)**

(54) **BASE MATERIALS FOR TISSUE REGENERATION, TRANSPLANT MATERIALS AND PROCESS FOR PRODUCING THE SAME**

BASISMATERIALIEN ZUR GEWEBEREGENERATION, TRANSPLANTATMATERIALIEN UND VERFAHREN ZUR HERSTELLUNG SELBIGER

MATERIAUX DE BASE POUR REGENERATION DE TISSUS, MATERIAUX DE TRANSPLANTATION, ET PROCEDE DE FABRICATION

(84) Designated Contracting States:
**DE FR GB IT**

(30) Priority: **03.07.2000 JP 2000201345**
**03.07.2000 JP 2000201346**

(43) Date of publication of application:
**04.06.2003 Bulletin 2003/23**

(73) Proprietors:
- **Japan Tissue Engineering Co., Ltd.**
  **Gamagori-shi,**
  **Aichi 443-0022 (JP)**
- **Yui, Nobuhiko**
  **Nomi-gun,**
  **Ishikawa-ken 923-1225 (JP)**

(72) Inventors:
- **YUI, Nobuhiko**
  **Nomi-gun,**
  **Ishikawa-ken 923-1225 (JP)**
- **OOYA, Tooru**
  **Ishikawa-gun,**
  **Ishikawa 920-2123 (JP)**
- **KATO, Masakazu,**
  **c/o Japan Tissue Eng. Co., Ltd.**
  **Gamagori-shi,**
  **Aichi 443-0022 (JP)**
- **YAMAMOTO, Takeyuki**
  **c/o Japan Tissue Eng. CO.,Ltd**
  **Gamagori-shi, Aichi 443-0022 (JP)**

(74) Representative: **Kuhnen, Rainer-Andreas**
**KUHNEN & WACKER**
**Patent- und Rechtsanwaltsbüro**
**Prinz-Ludwig-Strasse 40A**
**85354 Freising (DE)**

(56) References cited:
**WO-A1-96/09073** **JP-A- 9 263 547**
**JP-A- 9 301 893** **JP-A- 11 319 069**
**US-A- 6 037 387**

- **HARADA A: "Construction of supramolecular structures from cyclodextrins and polymers" CARBOHYDRATE POLYMERS, APPLIED SCIENCE PUBLISHERS, LTD. BARKING, GB, vol. 34, no. 3, 20 December 1997 (1997-12-20), pages 183-188, XP004101336 ISSN: 0144-8617**
- **WATANABE J ET AL: "Effect of acetylation of biodegradable polyrotaxanes on its supramolecular dissociation via terminal ester hydrolysis" JOURNAL OF BIOMATERIALS SCIENCE. POLYMER EDITION, VSP, UTRECHT, NL, vol. 10, no. 12, 1999, pages 1275-1288, XP002969569 ISSN: 0920-5063**
- **YUI N ET AL: "EFFECT OF BIODEGRADABLE POLYROTAXANES ON PLATELET ACTIVATION" BIOCONJUGATE CHEMISTRY, AMERICAN CHEMICAL SOCIETY, WASHINGTON, US, vol. 9, no. 1, 1998, pages 118-125, XP000729237 ISSN: 1043-1802**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- OOYA T ET AL: "Synthesis and characterization of biodegradable polyrotaxane as a novel supramolecular-structured drug carrier" JOURNAL OF BIOMATERIALS SCIENCE. POLYMER EDITION, VSP, UTRECHT, NL, vol. 8, no. 6, 1997, pages 437-455, XP002096596 ISSN: 0920-5063
- OOYA TOORU ET AL: "Polyrotaxanes: Synthesis, structure, and potential in drug delivery" CRITICAL REVIEWS IN THERAPEUTIC DRUG CARRIER SYSTEMS, vol. 16, no. 3, 1999, page 289, XP009045544 ISSN: 0743-4863
- OOYA T ET AL: "Biodegradable polyrotaxanes as a drug carrier" S.T.P. PHARMA SCIENCES 1999 FRANCE, vol. 9, no. 1, 1999, pages 129-138, XP009045547 ISSN: 1157-1489
- WATANABE J ET AL: "Feasibility study of hydrolyzable polyrotaxanes aiming at implantable materials" JOURNAL OF ARTIFICIAL ORGANS, SPRINGER VERLAG, TOKYO, JP, vol. 3, 2000, pages 136-142, XP002969568 ISSN: 1434-7229
- JUNJI WATANABE ET AL.: 'Soshiki kougaku wo mezashita seibunkai-sei polyrotaxane no sekkei' KOUBUNSHI KAKOU vol. 48, no. 5, 25 May 1999, pages 209 - 215, XP002936790

## Description

### Technical Field

The present invention relates to a base material for tissue reconstruction and an implantable material, which can be used widely in medical fields, such as orthopedic surgery, dental surgery, and plastic surgery, as well as to a method of preparing the implantable material.

### Background Art

Recently, tissue engineering to regenerate and reconstruct human tissues for treatment has been rapidly developed. Intensive studies have been performed in the field of tissue engineering since the early 1980s to culture skin cells, chondrocytes, and neurons in degradable or non-degradable materials for treatment of orthopedic surgery and the like.
There are some known treatment techniques in tissue engineering. One treatment technique implants a matrix functioning as a scaffold of tissue reconstruction to the living body and makes cells multiply in vivo. Another treatment technique cultures and multiplies cells on the matrix as the scaffold in vitro and implants the cultured cells (tissues) alone or with the matrix to the living body.
There are a diversity of known tissue-derived biomaterials and synthetic materials for the matrix functioning as the scaffold of tissue reconstruction. For example, the matrixes of collagen or polyglycolic acid allow for three-dimensional culture of cells and are decomposed and absorbed in vivo.
The decomposition products of such matrixes may, however, induce inflammation. Furthermore difficulties arise in regulation of the decomposition and disappearance time; for example, the matrix may be decomposed prior to reconstruction of the tissues or the matrix may not be decomposed even long after reconstruction of the tissues.
The object of the present invention is thus to solve the problems discussed above and to provide a base material for tissue reconstruction, which allows for tissue reconstruction and has physical properties required for tissue reconstruction as well as degradable and disappearing properties in vivo after reconstruction of the tissues. The object of the invention is also to provide an implantable material that allows for favorable tissue reconstruction and has degradable and disappearing properties in vivo after reconstruction of the tissues, and a method of preparing such an implantable material.

### Disclosure of the Invention

In order to solve the aforementioned problems, the present invention is a base material for tissue reconstruction that is mainly composed of a polyrotaxane hydrogel, where the polyrotaxane has biocompatible groups, each of which having a bulky substituent, that are introduced via hydrolysable in vivo bonds to either terminal of a linear molecule with multiple cyclic molecules threaded thereon, wherein the polyrotaxane hydrogel has a network structure formed by crosslinking between the cyclic molecules, between the biocompatible groups, or between the cyclic molecules and the biocompatible groups in adjoining polyrotaxane molecules.
Application of the base material for tissue reconstruction, which is mainly composed of the polyrotaxane hydrogel according to the present invention, to culture of chondrocytes causes the cells to multiply while maintaining the intrinsic characteristics of the chondrocytes. Implantation of the base material for tissue reconstruction alone in vivo does not interfere with the intrinsic properties or multiplication of the cells but ensures successful tissue reconstruction.
The polyrotaxane hydrogel used in the invention has physical properties required for tissue reconstruction. This may be ascribed to the high crystallinity of the polyrotaxane and the polyrotaxane hydrogel, due to their supramolecular structures, where different molecules, that is, linear molecules and cyclic molecules, have mechanical interlocking (non-covalent bonding).
The polyrotaxane hydrogel used in the invention also has degradable and disappearing properties in vivo after reconstruction of the tissues. This may be ascribed to its hydrolysable bondings. Hydrolysis of the hydrolysable bondings in vivo causes bulky biocompatible groups (that is, caps) to be detached from both terminals of the linear molecule. Detachment of the caps allows the cyclic molecules to be released from the linear molecule. This results in decomposition and disappearance of the polyrotaxane hydrogel.
As discussed above, the base material for tissue reconstruction according to the present invention allows for tissue reconstruction and has physical properties required for tissue reconstruction as well as degradable and disappearing properties in vivo after reconstruction of the tissues. This base material is thus suitable for reconstruction of the tissues from the cells.
In the base material for tissue reconstruction of the present invention, the linear molecule and the cyclic molecule are not specifically restricted as long as they have bio-compatibility (the properties that are substantially harmless to the living bodies). Preferably the linear molecule is one or more selected among the group consisting of polyethylene glycol, polypropylene glycol, copolymers of polyethylene glycol and polypropylene glycol, and polymethyl vinyl ether. Selection of the cyclic molecule and the linear molecule having excellent bio-compatibility enables the synthesized polyrotaxane or polyrotaxane hydrogel to exert the high bio-compatibility and the properties suitable for the implantable material for tissue reconstruction. The preferable average molecular weight of the linear molecule ranges from 200 to 50000, or more specifically 400 to 5000. Preferable examples of the cyclic molecule are α-, β-, and γ-cyclodextrins, although other compounds having analogous cyclic structures are also applicable. Examples of such compounds include cyclic polyethers, cyclic polyesters, cyclic polyether amines, and cyclic polyamines. A favorable combination of the linear molecule and the cyclic molecule is α-cyclodextrin and polyethylene glycol.

In the base material for tissue reconstruction of the present invention, the hydrolysable bond may be any bond that is hydrolyzed in vivo. A preferable example is an ester bond, which is quickly hydrolyzed in a non-enzymatic manner in vivo.

In the base material for tissue reconstruction is composed of the polyrotaxane hydrogel, the crosslinking is preferably any of a urethane bond, an amide bond, a carbamide bond, an ether bond, a sulfide bond, and a Schiff base linkage. In the case of crosslinking the cyclic molecules, it is preferable that the crosslinking is more stable to water than the hydrolysable bonding. The faster decomposition of the hydrolysable bonding causes the biocompatible groups having bulky substituents to be detached from both terminals of the linear molecule. The cross-linked cyclic molecules are then released at once. This gives a favorable decomposition pattern.

In the base material for tissue reconstruction of the present invention, the biocompatible groups on both terminals of the linear molecule may be any groups having high affinities to the living bodies (that is, groups having high safety to the living bodies). Preferable examples are amino acids, oligopeptides, oligosaccharides, and sugar derivatives. Typical examples of the amino acid include alanine, valine, leucine, isoleucine, methionine, proline, phenylalanine, tryptophan, aspartic acid, glutamic acid, glycin, serine, threonine, tyrosine, cysteine, lysine, arginine, and histidine. Typical examples of the oligopeptide include those formed by peptide linkage of a plurality of the amino acids selected among the above examples. Typical examples of the oligosaccharide include those having 1 to 5 repeating units and consisting of dextran, hyaluronic acid, chitin, chitosan, alginic acid, chondroitin sulfate, and starch as the polysaccharide constituent. Typical examples of the sugar derivative include chemically modified compounds, such as acetylated or isopropylated oligosaccharides, polysaccharides, and monosaccharides. Especially preferable are amino acids having benzene rings, such as L-phenylalanine, L-tyrosine, and L-tryptophan.

In the base material for tissue reconstruction of the present invention, the bulky substituent of the biocompatible group may be any group that can prevent the cyclic molecules from being released from the linear molecule. Preferable examples are groups having at least one benzene ring and groups having at least one tertiary butyl. Examples of the group having at least one benzene ring include benzyloxycarbonyl (Z) group, 9-fluorenylmethyloxycarbonyl (Fmoc) group, and benzyl ester (OBz) group. Examples of the group having at least one tertiary butyl include tertiary butylcarbonyl (Boc) group and amino acid-tertiary butyl ester (OBu) group. Especially preferable is benzyloxycarbonyl group.

In the base material for tissue reconstruction of the present invention, especially preferable are that the linear molecule is polyethylene glycol, the cyclic molecule is α-cyclodextrin, the hydrolysable bond is an ester bond, and the biocompatible group having the bulky substituent is benzyloxycarbonyl-L-phenylalanine. In the structure of threading α-cyclodextrin molecules onto the linear polyethylene glycol backbone, the stoichiometric ratio of α-cyclodextrin to the repeating units of ethylene glycols is 1 to 2.

The base material for tissue reconstruction of the present invention is composed of the polyrotaxane hydrogel, wherein the decomposition rate of the polyrotaxane hydrogel can be regulated by varying the weight percent of the polyrotaxane in the polyrotaxane hydrogel (that is, the weight ratio of the polyrotaxane to the polyrotaxane hydrogel). This is our new finding through the research. A preferable arrangement determines the weight percent of the polyrotaxane in the polyrotaxane hydrogel, based on this finding. The procedure specifies in advance a decomposition rate suitable for an application of the base material for tissue reconstruction and determines the weight percent of the polyrotaxane in the polyrotaxane hydrogel to attain the specified decomposition rate. This provides the base material for tissue reconstruction suitable for the application.

When the base material for tissue reconstruction of the present invention is composed of the polyrotaxane hydrogel, which has crosslinking between the cyclic molecules (between the biocompatible groups or between the cyclic molecule and the biocompatible group) in adjoining molecules of the polyrotaxane via a crosslinking linear molecule, the decomposition rate or the decomposition pattern of the polyrotaxane hydrogel can be regulated by varying the average molecular weight of the crosslinking linear molecule. This is also our new finding through the research. A preferable arrangement determines the average molecular weight of the crosslinking linear molecule, based on this finding. The procedure specifies in advance a decomposition rate or a decomposition pattern suitable for an application of the base material for tissue reconstruction and determines the average molecular weight of the crosslinking linear molecule to attain the specified decomposition rate or decomposition pattern. This provides the base material for tissue reconstruction suitable for the application.

The decomposition pattern may be a pattern of gradually decomposing in vivo or a pattern of abruptly decomposing in vivo after elapse of a certain time with substantially no decomposition. Preferable examples of the crosslinking linear molecule are biocompatible polymers, such as polyethylene glycol, polypropylene glycol, and copolymers of polyethylene glycol and polypropylene glycol. The average molecular weight is preferably in a range of 0 to 50000. Setting '0' to the molecular weight of the crosslinking means direct linkage without any crosslinking linear molecule. Crosslinking on both terminals of the crosslinking linear molecule is desirable.

The base material for tissue reconstruction of the present invention is a base material for tissue reconstruction that is mainly composed of a polyrotaxane hydrogel and has desirably characteristics (a) and/or (b) below, the polyrotaxane hydrogel having a network structure formed by crosslinking of cyclic molecules in adjoining molecules of a polyrotaxane via a crosslinking linear molecule, where the polyrotaxane has biocompatible groups, each of which has a bulky substituent and is introduced via a hydrolytic bond to either terminal of a linear molecule with multiple cyclic molecules threaded thereon:

(a) wherein a time for complete hydrolysis of the polyrotaxane hydrogel does not depend upon a water content in the polyrotaxane hydrogel, but is extended with any of a decrease in polyrotaxane content in the polyrotaxane hydrogel, an increase in molar ratio of the crosslinking linear molecule to the cyclic molecule, and a decrease in average molecular weight of the crosslinking linear molecule.

(b) wherein a decomposition pattern shown by a graph with $t/t_\infty$ as abscissa and $M_t/M_0$ as ordinate does not depend upon a content of the polyrotaxane in the polyrotaxane hydrogel but depends upon a molecular weight of the crosslinking linear molecule, where $t_\infty$ denotes a time for complete hydrolysis of the polyrotaxane hydrogel, t denotes a time elapsed, $M_0$ denotes an initial weight of the polyrotaxane hydrogel in a water-swelling state, and $M_t$ denotes a weight of the polyrotaxane hydrogel at the time t.

When the polyrotaxane hydrogel has the characteristics (a), the procedure specifies in advance a decomposition rate suitable for an application of the base material for tissue reconstruction and determines the percent (content) of the polyrotaxane in the polyrotaxane hydrogel, the molar ratio of the crosslinking linear molecule to the cyclic molecule, or the average molecular weight of the crosslinking linear molecule to attain the specified decomposition rate. This provides the base material for tissue reconstruction suitable for the application.

When the polyrotaxane hydrogel has the characteristics (b), on the other hand, the procedure specifies in advance a decomposition pattern suitable for an application of the base material for tissue reconstruction and determines the average molecular weight of the crosslinking linear molecule to attain the specified decomposition pattern. This provides the base material for tissue reconstruction suitable for the application. The average molecular weight of the crosslinking linear molecule is preferably in a range of 4000 to 10000 to attain the pattern of abruptly decomposing in vivo after elapse of a certain time with substantially no decomposition.

Typical examples of the base material for tissue reconstruction having the characteristics (a) and/or (b) are that the cyclic molecule is α-cyclodextrin, the linear molecule is polyethylene glycol, the hydrolytic bond is an ester bond, the crosslinking linear molecule is polyethylene glycol bis-amine, and the polyrotaxane hydrogel has a network structure formed by crosslinking OH groups of the α-cyclodextrins included in adjoining molecules of the polyrotaxane via urethane bonds of $NH_2$ groups on both terminals of the polyethylene glycol bis-amine.

The base material for tissue reconstruction of the present invention may be applied in any suitable forms that allow culture or incorporation of cells. For example, cells may be seeded on a film of the base material, on a gel of the base material, in a solution of the base material, or in a suspension of the base material. Because of its properties of readily holding and culturing cells, a porous structure of the polyrotaxane or the polyrotaxane hydrogel is desirable. The pores of the porous structure are not specifically restricted but should have the suitable size and density to hold the cells therein. When the base material for tissue reconstruction is not combined with cells but is implanted alone in vivo, there is no limitation in the form of the base material for tissue reconstruction. The porous structure is, however, preferable since it provides the desirable environment for multiplication of cells from the peripheral tissues about the implanted piece. The pores of the porous structure are not specifically restricted but should have the suitable size and density to allow invasion of cells from the peripheral tissues about the implanted piece and vascularization. Any known technique is applicable to produce the porous structure: for example, the gelation in the presence of sodium chloride or the freeze-dry lyophilization of the water-containing hydrogel.

The base material for tissue reconstruction of the present invention may have surface coated with a cell adhesion promoting substance. The cell adhesion promoting substance is any substance having the property of promoting adhesion of cells. Typical examples are collagen and gelatin. The coating method is not specifically restricted but may be any conventional procedure. A simple process prepares a porous structure, soaks the porous structure in the cell adhesion promoting substance, and freeze-dries the soaked porous structure.

The base material for tissue reconstruction may be used for culture or incorporation of any cells. For example, the base material is applied for culture of mesenchymal cells, hepatic cells, and neurons. The mesenchymal cells include chondrocytes, fibroblasts, osteoblasts, myoblasts, lipocytes, endothelial cells, epithelial cells, and their precursor cells. The base material for tissue reconstruction of the present invention is effectively applied for culture of chondrocytes.

In order to attain reconstruction of tissues with the base material for tissue reconstruction of the present invention, the procedure may apply the polyrotaxane or the polyrotaxane hydrogel alone, may simply incorporate the cells into the base material for tissue reconstruction, or may multiply the cells in the base material for tissue reconstruction.

Various techniques are applicable for fixation of cells. One available procedure for fixation adds a high concentration of a cell suspension to the polyrotaxane hydrogel and makes the cells incorporated into the gel pores with swelling of the gel. Another available procedure for fixation is rotary culture. Still another available procedure for fixation seeds cells and then reduces the pressure to a specific level that does not significantly affect the cells.

An implantable material including the base material for tissue reconstruction of the present invention with cells cultured thereon or incorporated therein allows for reconstruction of tissues. Any methods are applicable to prepare the implantable material. A preferable procedure provides an adequate size and shape of the base material for tissue reconstruction according to the application and subsequently causes cells to be cultured on or incorporated in the base material for tissue reconstruction to obtain the implantable material. For example, when the implantable material is applied for reconstruction of cartilage of ear, the procedure shapes and processes the base material for tissue reconstruction according to the target site of the ear, injects a cell suspension into the processed base material for tissue reconstruction, and cultures the cells for a preset time period to obtain the implantable material. The implantable material thus obtained is embedded in the target site of the ear. Another available procedure first cultures or incorporates cells on or into the base material for tissue reconstruction to obtain an implantable material and shapes and processes the implantable material to an adequate size and shape according to the target site at the time of actual application or at the time of delivery.

In the implantable material where the chondrocytes are cultured on the base material for tissue reconstruction, which is composed of the polyrotaxane or the polyrotaxane hydrogel, the cultured cells are multiplied and vigorously produce a cartilage substrate while maintaining the intrinsic characteristics of the chondrocytes. The cartilage tissue is mainly repaired with the chondrocytes and the substrate produced by the chondrocytes. The high content of the chondrocytes and the cartilage substrate means that the implantable material has high tissue reconstructing ability. The advantages of the culture are that the cells required for repairing the tissues are multiplied and that the products of the cells (the substrate and the growth factor) are carried on the implantable material. Even if cells are annihilated by some reason, the substrate and the growth factor produced by the cells are left in the implantable material to effectively function for reconstruction of tissues. In the case where the chondrocytes are simply seeded on the base material for reconstruction, that is, when the chondrocytes are simply incorporated in the base material without multiplication, the incorporated cells maintain their intrinsic properties and start functioning to reconstruction of the tissues immediately after implantation. This arrangement thus also provides the effective implantable material.

## Brief Description of the Drawings

Fig. 1 shows a process of synthesizing a biodegradable polyrotaxane;
Fig. 2 shows a process of synthesizing a biodegradable polyrotaxane hydrogel;
Fig. 3 is a graph showing non-enzymatic hydrolytic behaviors of polyrotaxane hydrogels PRHG-5,6;
Fig. 4 is a graph showing decomposition patterns of the polyrotaxane hydrogels PRHG-5,6;
Fig.5 is a graph showing non-enzymatic hydrolytic behaviors of polyrotaxane hydrogels PRHG-7 through 9;
Fig. 6 is a graph showing decomposition patterns of the polyrotaxane hydrogels PRHG-7 through 9;
Fig. 7 is graphs showing decomposition behaviors of the polyrotaxane hydrogels with variations in content of the polyrotaxane, under the condition of a fixed molecular weight of the PEG bis-amine;
Fig. 8 is a graph showing the relationship between the content of the polyrotaxane and the complete decomposition time;
Fig. 9 is a graph showing the relationship between the content of the polyrotaxane and the water content;
Fig. 10 is a graph showing the complete decomposition time plotted against the PEG/$\alpha$-CD ratio;
Fig. 11 is graphs showing decomposition patterns with $t/t_\infty$ as abscissa and $M_t/M_0$ as ordinate;
Fig. 12 shows variations in culture of rabbit chondrocytes on polyrotaxane hydrogels PRHG-1,3 over time.

## Best Modes of Carrying Out the Invention

Preferable examples of the invention are discussed below, although the invention is not at all restricted to these examples.

(1) Synthesis of Biodegradable Polyrotaxane (see Fig. 1)

(1-1) Synthesis of PEG having amino groups on both terminals

Polyethylene glycol (PEG) (33g, 10 mmol) having a molecular weight of 3,300 and succinic anhydride (20 g, 200 mmol) were dissolved in toluene (220 ml). The resulting solution was refluxed at 150°C for 5 hours. On completion of the reaction, the solution was poured into an excess of diethyl ether, was filtered, and was dried under reduced pressure. A crude product thus obtained was dissolved in dichloromethane. After removal of an insoluble substance by centrifugation, the solution was poured into an excess of diethyl ether, was filtered, and was dried under reduced pressure. This gave the PEG having carboxyl groups on both terminals thereof (compound A) as white powder.

The compound A (20 g, 5.7 mmol) and N-hydroxysuccinimide (HOSu) (17.1 g, 148.2 mmol) were dissolved in a mixed solution of 1,4-dioxane and dichloromethane (350 ml, volume ratio 1:1). After cooling of the solution with ice, dicyclohexylcarbodiimide (DCC) (23.5 g, 114 mmol) was added to the solution. The resulting solution was stirred for 1 hour while being cooled with ice, and further stirred overnight at room temperature. After dicyclourethane as a by-product was filtered out, the filtrate was concentrated, was poured into an excess of diethyl ether, was filtered, and was dried under reduced pressure. This gave the PEG with activated carboxyl groups (compound B) as white powder.

A dichloromethane solution (75 ml) of the compound B (10 g, 2.7 mmol) was added dropwise to a dichloromethane (75 ml) solution of ethylenediamine (0.4 ml, 6 mmol). After conclusion of the dropwise addition, the solution mixture was stirred for 1 hour at room temperature. On completion of the reaction, the solution was poured into an excess of diethyl ether, was filtered, and was dried under reduced pressure. This gave the PEG having amino groups on both terminals thereof (compound C) as white powder.

(1-2) Preparation of Pseudopolyrotaxane

An aqueous solution (20 ml) of the compound C (4 g, 1.12 mmol) was added dropwise to a saturated aqueous solution (311 ml) of $\alpha$-cyclodextrin ($\alpha$-CD) (48 g, 49.2 mmol) at room temperature. The resulting solution was stirred for 1 hour with irradiation of ultrasonic waves, and was further stirred for 24 hours at room temperature. A white precipitate was recovered by centrifugation, and was dried under reduced pressure at 50°C. This gave white powder of pseudopolyrotaxane.

The polyrotaxane has a large number of cyclic molecules (for example, cyclodextrin) threaded on a linear molecule (for example, PEG) and bulky substituents for capping on both terminals of the linear molecule. The pseudopolyrotaxane does not have any bulky substituents for capping on both terminals of the polyrotaxane.

(1-3) Preparation of Terminal Capping Component

For introduction of benzyloxycarbonyl-L-phenylalanine (Z-L-Phe: Z denotes a benzyloxycarbonyl group) as the bulky substituents for preventing desorption of $\alpha$-CD, the carboxyl group of Z-L-Phe was activated.

Z-L-Phe (100 g, 334 mmol) was dissolved in 1,4-dioxane (800 ml). HOSu (38.42 g, 334 mmol) was added to the solution while being cooled with ice. After 1 hour, a 1,4-dioxane solution (200 ml) of DCC (75.7 g, 367 mmol) was added slowly to the solution. The resulting solution was stirred for 1 hour while being cooled with ice, and was further stirred overnight at room temperature. After dicyclourethane as the by-product was filtered out, the filtrate was concentrated, was poured into an excess of diethyl ether, was filtered, and was dried under reduced pressure. This gave a crude product. The crude product was dissolved in dichloromethane at room temperature to a level closest to its saturated concentration. After addition of an adequate quantity of petroleum ether, the solution was kept in cold for recrystallization. White needle crystal, a succinimide ester of Z-L-Phe (Z-L-Phe-OSu), was obtained after filtration and drying under reduced pressure.

(1-4) Preparation of Biodegradable Polyrotaxane

The Z-L-Phe-OSu (80 g, 200 mmol) was dissolved in dimethyl sulfoxide (DMSO) (60 ml), and the pseudopolyrotaxane (45 g, 2 mmol) was added to the solution. DMSO was added little by little to this heterogeneous solution at room temperature with stirring for 96 hours to the homogeneous state. On completion of the reaction, the reaction solution was poured into an excess of diethyl ether. This gave a crude product. The crude product was washed successively with acetone and dimethylformamide (DMF) for removal of impurities (including non-reacted Z-L-Phe-OSu, $\alpha$-CD, and the compound C). A biodegradable polyrotaxane as white powder was obtained after filtration and drying under reduced pressure. The result of the synthesis was checked by $^1$H-NMR.

(2) Preparation of Polyrotaxane Hydrogels (see Fig. 2)

The biodegradable polyrotaxane (1 g, $2.91\times10^{-5}$ mol, [OH] =16.2 mmol) was dissolved in DMSO (10 ml) in a nitrogen atmosphere. After addition of N,N'-carbonyldimidazole (CDI) (8.13 mmol), the solution was stirred for 3 hours at room temperature. On completion of the reaction, the solution was poured into an excess of diethyl ether, was filtered, and was dried under reduced pressure. This gave white powder of CDI activated polyrotaxane (CDI-PR).

A DMSO solution of CDI-PR, PEG bis-amine (PEG-BA, molecular weight: 600), and sodium chloride were mixed together according to the compositions shown in Table 1 given below. After stirring and deaeration, the mixtures were subjected to gelation with Teflon spacers (diameter: 13 mm, depth: 2 mm) at 35°C for 24 hours. The resulting gels were washed first with DMSO and subsequently with water until no variation in weight. This gave polyrotaxane hydrogels (PRHG-1 through PRHG-4).

Each of these polyrotaxane hydrogels PRHG-1 through PRHG-4 has a three-dimensional network structure, in which the cyclic molecules (α-cyclodextrin) included in adjoining molecules of the polyrotaxane are bridged via PEG by a crosslinking bond (urethane bond). The hydrogels PRHG-1 through PRGH-3 gelated in the presence of sodium chloride were porous bodies (checked by electron microscope), while the hydrogel PRHG-4 gelated in the absence of sodium chloride was a non-porous structure.

[Table 1]

| | | PRHG-1 | PRHG-2 | PRHG-3 | PRHG-4 |
|---|---|---|---|---|---|
| Reaction Conditions for Hydrogelation | Concentration of CDI-PR(g/ml) in reaction solution. The value in [ ] shows the Prconverted concentration(g/ml). | 0.30 [0.20] | 0.20 [0.12] | 0.20 [0.12] | 0.30 [0.20] |
| | Concentration of PEG-BA(g/ml) in the reaction solution. The value in < > shows the molecular weight of PEG-BA. | 0.33 <0.6K> | 0.26 <2.0K> | 0.26 <0.6K> | 0.09 <0.6K> |
| | Amount of NaCl used (% by weight to CDI-PR+PEG-BA) | 38 | 100 | 100 | 0 |
| Content of Polyrotaxane (wt%) in Hydrogel | | 38 | 32 | 32 | 70 |
| Properties of hydrogel*) | Water Content(wt%) | 87.1 | - | 97.1 | - |
| | Compressive Modulus($\times10^4$ Pa) | 6.49 | - | 1.44 | - |
| | Crosslinking Density($\times10^{-6}$ mol/cm$^3$ ) | 8.72 | - | 1.93 | - |

*) Observed values with regard to the non-porous bodies

(3) Properties of Polyrotaxane Hydrogels

The test measured the water content, the compressive modulus of elasticity as an index of dynamic strength, and the crosslinking density in the swelling state of the polyrotaxane hydrogels (see Table 1). The values in Table 1 are the observed values with regard to the non-porous bodies. The porous structure has the compressive modulus of elasticity equivalent to or smaller by one order than that of the non-porous structure, and the crosslinking density equivalent to that of the non-porous structure.

The test measured a weight ($W_{wet}$) of the polyrotaxane hydrogel in the equilibrium swelling state at room temperature and a weight ($W_{dry}$) of the dried polyrotaxane hydrogel after drying of the gel under reduced pressure at 50°C, and calculated the water content according to Equation 1 given below:

[Equation 1]

$$\text{Water Content (\%)} = \frac{W_{wet} - W_{dry}}{W_{wet}} \times 100$$

The compressive modulus of elasticity was measured at room temperature with a heat stress-induced strain measurement device. The crosslinking density was calculated from the observed compressive modulus of elasticity according to Equation 2 given below. The measurement used the polyrotaxane hydrogels in the equilibrium swelling state, which were punched out by means of a cork borer to a substantially identical cross section with that of a probe.

[Equation 2]

$$Ec = 3RT \times ve/V$$

Where Ec denotes the compressive modulus of elasticity, ve/V denotes the crosslinking density,
R denotes the gas constant,
T denotes the absolute temperature, and
V denotes the volume of the gel in the swelling state.

(4) Analysis of Non-enzymatic Hydrolytic Behaviors of Polyrotaxane Hydrogels

(4-1) Discussion on Percent of Polyrotaxane in Polyrotaxane Hydrogel

The gelation process (2) of the mixture containing the DMSO solution of CDI-PR, the DMSO solution of PEG-BA, and sodium chloride was performed to prepare a polyrotaxane hydrogel (PRHG-5) where the ratio of the weight of the polyrotaxane (converted from the DMSO solution of CDI-PR) to the weight of PEG-BA was 70 to 30, and a polyrotaxane hydrogel (PRHG-6) where the weight ratio was 60 to 40.

The polyrotaxane hydrogels PRHG-5 and PRHG-6 in the equilibrium swelling state were respectively soaked in a 0.1 M phosphate buffer solution (PBS) (pH =8.0) and were shaken at 37°C. The variation in weight of each hydrogel was measured, while water was wiped off at preset time intervals. The weight variation was used for evaluation of the non-enzymatic hydrolytic behavior of the hydrogel. The results of the measurement are shown in Fig. 3.

In the decomposition pattern of the polyrotaxane hydrogel, the weight of the polyrotaxane hydrogel initially increases and then decreases to zero. During the increase in weight, the number of crosslinking points in the polyrotaxane hydrogel decreases to expand the size of each grid in the three-dimensional network structure, while the three-dimensional structure of the polyrotaxane hydrogel is maintained. This facilitates storage of water and raises the water content. When the number of crosslinking points further decreases to destroy the three-dimensional structure of the polyrotaxane hydrogel, however, the decomposition of the polyrotaxane hydrogel decreases the weight to zero. The time period between the time when measurement of the weight variation starts and the time when the weight reaches zero relates to the decomposition rate.

As shown in Fig. 3, the hydrogel PRHG-5 has the higher decomposition rate than that of the hydrogel PRHG-6. Namely the greater weight percent of the polyrotaxane in the polyrotaxane hydrogel results in the higher decomposition rate of the polyrotaxane hydrogel.

For further discussion, the decomposition pattern of the polyrotaxane hydrogel was standardized with a weight $M_0$ of the polyrotaxane hydrogel in the water-swelling state before the start of the experiment and a time $t_\infty$ for complete decomposition of the polyrotaxane hydrogel (hereafter referred to as the complete decomposition time). The result of the standardization is shown in the graph of Fig. 4, where $t/t_\infty$, which is a division of a time t elapsed since soaking of the polyrotaxane hydrogel in water by the time $t_\infty$, is plotted against $M_t/M_0$, which is a division of a weight Mt of the polyrotaxane hydrogel at the time t by the weight $M_0$. One of the critical points in the decomposition pattern is the value of $t/t_\infty$, at which decomposition of the polyrotaxane hydrogel starts (at which $M_t/M_0$ reaches a peak). The smaller value of $t/t_\infty$ at the start of decomposition means the longer time period required for complete decomposition since the start of decomposition. On the contrary, the greater value of $t/t\infty$ at the start of decomposition means the shorter time period required for complete decomposition since the start of decomposition.

As shown in Fig. 4, both the hydrogels PRHG-5 and PRHG-6 have substantially identical values (about 0.5) of $t/t_\infty$ at the start of decomposition. This accordingly proves that the decomposition pattern of the polyrotaxane hydrogel does not depend upon the percent of the polyrotaxane in the polyrotaxane hydrogel.

(4-2) Discussion on Molecular Weight of Crosslinking Portion in Polyrotaxane Hydrogel

The gelation process (2) of the mixture containing the DMSO solution of CDI-PR, the DMSO solution of PEG-BA, and sodium chloride was performed to prepare a polyrotaxane hydrogel (PRHG-7) where the PEG-BA used had a molecular weight of 600, a polyrotaxane hydrogel (PRHG-8) where the PEG-BA used had a molecular weight of 2000, and a polyrotaxane hydrogel (PRHG-9) where the PEG-BA used had a molecular weight of 4000, while the mixing ratio of PEG-BA to the $\alpha$-CD content in CDI-PR was set equal to 1.0.

The polyrotaxane hydrogels PRHG-7, PRHG-8, and PRHG-9 in the equilibrium swelling state were respectively soaked in a 0.1 M phosphate buffer solution (PBS) (pH =7.4) and were shaken at 37°C. The variation in weight of each hydrogel was measured, while water was wiped off at preset time intervals. The weight variation was used for evaluation of the non-enzymatic hydrolytic behavior of the hydrogel. The results of the measurement are shown in Fig. 5.

The result of Fig. 5 shows that the smaller molecular weight of the crosslinking portion in the polyrotaxane hydrogel leads to the higher decomposition rate of the polyrotaxane hydrogel. This means that the decomposition rate of the polyrotaxane hydrogel is regulated by varying the molecular weight of the crosslinking portion in the polyrotaxane hydrogel.

For further discussion, like the graph of Fig. 4, the decomposition pattern of the polyrotaxane hydrogel was standardized in the graph of Fig. 6. As shown in Fig. 6, the value of $t/t_\infty$ at the start of decomposition was about 0.50 for the hydrogel PRHG-7, about 0.85 for the hydrogel PRHG-8, and about 0.90 for the hydrogel PRHG-9. This shows that the greater molecular weight of the crosslinking portion in the polyrotaxane hydrogel leads to the greater value of $t/t_\infty$ at the start of decomposition in the decomposition pattern of the polyrotaxane hydrogel.

(4-3) Conclusions

As clearly understood from the above results, the decomposition rate of the polyrotaxane hydrogel is regulated by varying the weight percent of the polyrotaxane in the polyrotaxane hydrogel or by varying the molecular weight of the crosslinking portion in the polyrotaxane hydrogel. In the case where the polyrotaxane hydrogel is used as a base material for tissue reconstruction, the process specifies the decomposition rate of the polyrotaxane hydrogel suitable for an application of the base material for tissue reconstruction, and determines the weight percent of the polyrotaxane in the polyrotaxane hydrogel or the molecular weight of the crosslinking portion in the polyrotaxane to attain the specified decomposition rate.

The decomposition pattern of the polyrotaxane hydrogel ($t/t_\infty$ at the start of decomposition) is controllable by varying the molecular weight of the crosslinking portion in the polyrotaxane hydrogel. In the case where the polyrotaxane hydrogel is used as the base material for tissue reconstruction, the process specifies a decomposition pattern suitable for an application of the base material for tissue reconstruction, and determines the molecular weight of the crosslinking portion in the polyrotaxane hydrogel to attain the specified decomposition pattern.

(5) Analysis of Non-enzymatic Hydrolytic Behaviors of Polyrotaxane Hydrogels - Part 2

The CDI-PR ($9.2\times10^{-5}$ mol, N-acylcarbonate group: 10.7 mmol) was dissolved in DMSO (5 ml). Various molten PEG bis-amines were added to the solution. Table 2 shows a list of the synthesis conditions. Each reaction mixture was deaerated, was injected to Teflon spacers, and was kept at 35°C for 24 hours. The resulting product was washed with DMSO and was soaked in water at room temperature for 2 days for complete hydration. This gave polyrotaxane hydrogels.

[Table 2]

| *1) Code | Molecular weight of PEG bis-amine | Content of polyrotaxane (wt%) | PEG/α-CD*2) | Percentage of content (%) |
|---|---|---|---|---|
| E4/RX47 | 4000 | 47 | 0.34 | 77.6 |
| E4/RX35 | 4000 | 35 | 0.54 | 79.6 |
| E9/RX29 | 4000 | 29 | 0.71 | 80.9 |
| E4/RX26 | 4000 | 26 | 0.84 | 81.6 |
| E4/RX21 | 4000 | 21 | 1.11 | 82.2 |
| E2/RX81 | 2000 | 81 | 0.14 | 84.8 |
| E2/RX63 | 2000 | 63 | 0.35 | 83.9 |
| E2/RX52 | 2000 | 52 | 0.54 | 85.4 |
| E2/RX44 | 2000 | 44 | 0.76 | 85.4 |
| E2/RX37 | 2000 | 37 | 0.94 | 85.9 |
| E0.6/RX85 | 600 | 85 | 0.36 | 60.5 |
| E0.6/RX79 | 600 | 79 | 0.53 | 57.6 |
| E0.6/RX73 | 600 | 73 | 0.74 | 57.8 |
| E0.6/RX67 | 600 | 67 | 0.98 | 60.5 |

*1) The figure after 'E' represents 1/1000 of the average molecular weight of PEG bis-amine. The figure after 'RX' represents the content(% by weight) of the polyrotaxane.
*2) PEG/α-CD represents the calculated molar ratio of PEG bis-amine to α-CD in the polyrotaxane hydrogel.

The hydrolytic behaviors were examined in the following manner. Each of the swelling polyrotaxane hydrogels thus obtained was cut into 1 cm × 1 cm slabs (thickness < 1 mm). The slabs were soaked at 37°C in a 0.1 M phosphate buffer (pH 7.4) containing 0.02% sodium azide, and were shaken at 130 rpm by a shaker. Decomposition of the polyrotaxane hydrogel was evaluated by measuring the weight of the remaining polyrotaxane hydrogel. The experiment was repeated three times. The results of the experiment are shown in the graphs of Figs. 7 through 9.

The graphs of Fig. 7 show decomposition behaviors of the polyrotaxane hydrogels with variations in content of the polyrotaxane, under the condition of a fixed molecular weight of the PEG bis-amine. Figs. 7(a) through 7(c) respectively show the results for the PEG bis-amines having the molecular weight of 4000, 2000, and 600. The graph of Fig. 8 shows the relationship between the content of the polyrotaxane and the complete decomposition time. As clearly understood from the graphs of Figs. 7 and 8, the decrease in content of the polyrotaxane results in the longer complete decomposition time, under the condition of a fixed molecular weight of the PEG bis-amine. Namely the greater weight percent of the polyrotaxane in the polyrotaxane hydrogel leads to the higher decomposition rate of the polyrotaxane hydrogel.

The graph of Fig. 9 shows the relationship between the content of the polyrotaxane and the water content. As clearly understood from the graph of Fig. 9, the water content in the polyrotaxane hydrogel is practically constant regardless of the content of the polyrotaxane, under the condition of a fixed molecular weight of the PEG bis-amine. This result shows that the water content is not the factor of varying the complete decomposition time.

Fig. 10 is a graph showing the complete decomposition time plotted against the PEG/α-CD ratio. Here the PEG/α-CD ratio represents the molar ratio of PEG bis-amine to α-CD in the polyrotaxane hydrogel. As clearly understood from the graph of Fig. 10, the greater PEG/α-CD ratio results in the longer complete decomposition time, under the condition of a fixed molecular weight of the PEG bis-amine. Namely the smaller PEG/α-CD ratio leads to the higher decomposition rate of the polyrotaxane hydrogel. As shown in Table 2, the PEG/α-CD ratio was adjusted to be not greater than a value '2'. When the PEG/α-CD ratio exceeds the value '2', the polyrotaxane hydrogel is not decomposed under physiological conditions or in a 0.1 M aqueous solution of sodium hydroxide. The PEG/α-CD ratio was accordingly set to be not greater than the value '2'.

Like Fig. 4, the graphs of Fig. 11 show decomposition patterns with $t/t_\infty$ as abscissa and $M_t/M_0$ as ordinate. As clearly understood from the graphs of Fig. 11, the decomposition patterns do not depend upon the content of the polyrotaxane in the polyrotaxane hydrogel but have similar profiles under the condition of a fixed molecular weight of the PEG bis-amine. The time lag to the start of decomposition of the polyrotaxane hydrogel is lengthened with an increase in molecular weight of the PEG bis-amine. These results show that the greater molecular weight of the PEG bis-amine results in the longer time lag to the start of decomposition and gives a pattern of early decomposition.

(6) Culture of Chondrocytes

The polyrotaxane hydrogels PRHG-1 and PRHG-3 shown in Table 1 were autoclaved and sterilized at 121°C for 20 minutes. Each of the sterilized polyrotaxane hydrogels was set in a 96 well culture plate. Rabbit chondrocytes cryopreserved (kept in a frozen state) and thawed were seeded at a concentration of $2\times10^5$ cells / 100 μl per well and were stood still at 37°C in a 5% $CO_2$ incubator. This caused fixation of the cells to each polyrotaxane hydrogel (fixation time: 2 hours or 24 hours).

Each polyrotaxane hydrogel with the chondrocytes fixed thereto was transferred to a 24 well culture plate. A Dulbecco modified Eagle's medium (DMEM) containing 10 v/v% fetal bovine serum (FBS) and 50 μg/ml ascorbic acid (hereafter simply referred to as the medium) was added to the culture plate. The medium was replaced at every 7 days. The total culture time was 6 weeks. The culture was fixed with 10% formalin, was dyed with Alcian Blue (pH 1.0), and was sealed after decoloration, dehydration, and penetration. The Alcian Blue dyeing is one of the techniques for dyeing acidic mucopolysaccharides and is applicable to dye the cartilage tissue.

In the case where the fixation time was 2 hours, round cells started multiplication in a colony immediately after the start of the culture, and the colony grew over the time of culture. The growth of the colony was observed at the culture day 14, the culture day 28, and the culture day 42 (see Fig. 12). The colony produced somewhat opaque substrate with its growth. This gave cloudy images under microscope. After the 4-week culture, distinct Alcian Blue-positive images were observed over the whole cell colony. Namely the application of the polyrotaxane hydrogel for the culture caused the chondrocytes to multiply and produce the substrate (acidic mucopolysaccharides) while maintaining the intrinsic round shape. The three-dimensional cultures of the chondrocytes on the base materials for tissue reconstruction composed of the polyrotaxane hydrogels (PRHG-1 and PRHG-3) can thus be used as implantable materials. Similar results were obtained in the case where the fixation time was 24 hours.

The colony of the chondrocytes was spilled into the culture plate and adhered to the bottom of the culture plate, with the extension of cells or with the decomposition of the polyrotaxane hydrogel. Fibroblast-like cells were then grown out of the adhesive colony. These outgrowth cells did not hold the intrinsic round shape of the chondrocytes and were negative in Alcian Blue dyeing. These results may prove dedifferentiation, since monolayer culture of the colony of the chondrocytes kept in the polyrotaxane hydrogel caused the cultured cells to loose the intrinsic characters of the chondrocytes.

## Industrial Applicability

The present invention provides a base material for tissue reconstruction and an implantable material, which can be used widely in medical fields, such as orthopedic surgery, dental surgery, and plastic surgery.

## Claims

1. A base material for tissue reconstruction that is mainly composed of a polyrotaxane hydrogel, where the polyrotaxane has biocompatible groups, each of which having a bulky substituent, that are introduced via hydrolysable in vivo bonds to either terminal of a linear molecule with multiple cyclic molecules threaded thereon, wherein the polyrotaxane hydrogel has a network structure formed by crosslinking between the cyclic molecules, between the biocompatible groups, or between the cyclic molecules and the biocompatible groups in adjoining polyrotaxane molecules.

2. A base material for tissue reconstruction in accordance with claim 1, wherein the linear molecule is any of polyethylene glycol, polypropylene glycol, copolymers of polyethylene glycol and polypropylene glycol, and polymethyl vinyl ether, and the cyclic molecule is any of α-, β-, and γ-cyclodextrins.

3. A base material for tissue reconstruction in accordance with either one of claims 1 and 2, wherein the hydrolysable in vivo bonds are ester bonds.

4. A base material for tissue reconstruction in accordance with claim 3, wherein the crosslinking is any of urethane bonds, amide bonds, carbamide bonds, ether bonds, sulfide bonds, and Schiff base linkages.

5. A base material for tissue reconstruction in accordance with any one of claims 1 through 4, wherein the bulky substituent is either one of a group having at least one benzene ring and a group having at least one tertiary butyl.

6. A base material for tissue reconstruction in accordance with any one of claims 1 through 5, wherein the biocompatible groups are any of amino acids, oligopeptides, oligosaccharides, sugar derivatives.

7. A base material for tissue reconstruction in accordance with any one of claims 1 through 6, wherein the linear molecule is polyethylene glycol, the cyclic molecules are α-cyclodextrins, the hydrolysable in vivo bonds are ester bonds, the biocompatible groups having the bulky substituents are benzyloxycarbonyl-L-phenylalanines.

8. A base material for tissue reconstruction in accordance with any one of claims 1 through 7, wherein the polyrotaxane hydrogel has a porous structure.

9. A base material for tissue reconstruction in accordance with any one of claims 1 through 8, wherein the polyrotaxane hydrogel has the crosslinking between the cyclic molecules, between the biocompatible groups, or between the cyclic molecules and the biocompatible groups via crosslinking linear molecules, and wherein the polyrotaxane hydrogel has the characteristics (a):

(a) wherein a time for complete hydrolysis of the polyrotaxane hydrogel ($t_\infty$) is independent upon a water content in the polyrotaxane hydrogel, but is extended with any of a decrease in polyrotaxane content in the polyrotaxane hydrogel, an increase in molar ratio of the crosslinking linear molecules to the cyclic molecules, and a decrease in average molecular weight of the crosslinking linear molecules.

10. A base material for tissue reconstruction in accordance with any one of claims 1 through 8, wherein the polyrotaxane hydrogel has the crosslinking between the cyclic molecules, between the biocompatible groups, or between the cyclic molecules and the biocompatible groups via crosslinking linear molecules, and wherein the polyrotaxane hydrogel has the characteristics (b):

(b) wherein a decomposition pattern presented as a graph with $t/t_\infty$ as abscissa and $M_t/M_0$ as ordinate, where $t_\infty$ is a time for complete hydrolysis of the polyrotaxane hydrogel, t is a time for soaking of the polyrotaxane hydrogel in water, $M_0$ is an initial weight of the polyrotaxane hydrogel in a water-swelling state, and $M_t$ is a weight of the polyrotaxane hydrogel at the time t, is independent upon a content of the polyrotaxane in the polyrotaxane hydrogel, but is dependent upon a molecular weight of the crosslinking linear molecules.

11. A base material for tissue reconstruction in accordance with either one of claims 9 and 10, wherein the cyclic molecules are α-cyclodextrins, the linear molecule is polyethylene glycol, the hydrolysable in vivo bonds are ester bonds, the crosslinking linear molecules are polyethylene glycol bis-amines, and wherein the polyrotaxane hydrogel has a network structure, in which OH groups of the α-cyclodextrins included in adjoining polyrotaxane molecules are crosslinked via urethane bonds of $NH_2$ groups on both terminals of the polyethylene glycol bis-amines.

12. A base material for tissue reconstruction in accordance with any one of claims 1 through 11, for the use for culture of any of mesenchymal cells, hepatic cells, and neurons.

13. An implantable material, comprising cells cultured on or incorporated in a base material for tissue reconstruction in accordance with any one of claims 1 through 12.

14. A method of preparing an implantable material, said method providing an implantable material by making cells cultured on or incorporated in a base material for tissue reconstruction in accordance with any one of claims 1 through 12.

**Patentansprüche**

1. Ausgangsmaterial zum Wiederaufbau von Gewebe, das hauptsächlich aus einem Polyrotaxan-Hydrogel besteht, wobei das Polyrotaxan biokompatible Gruppen mit jeweils einem sperrigen Substituenten aufweist, die durch hydrolysierbare *in vivo* Bindungen an eines von beiden Enden eines linearen Moleküls mit zahlreichen darauf aufgefädelten cyclischen Molekülen eingeführt werden und worin das Polyrotaxan-Hydrogel eine Netzwerkstruktur aufweist, die durch Vernetzung zwischen den cyclischen Molekülen, zwischen den biokompatiblen Gruppen oder zwischen den cyclischen Molekülen und den biokompatiblen Gruppen in benachbarten Polyrotaxanmolekülen gebildet wird.

2. Ausgangsmaterial zum Wiederaufbau von Gewebe nach Anspruch 1, worin das lineare Molekül ein beliebiges von Polyethylenglycol, Polypropylenglykol, Copolymeren von Polyethylenglycol und Polypropylenglykol, und Polymethylvinylether ist, und das cyclische Molekül ein beliebiges von α-, β-, und γ-Cyclodextrinen ist.

3. Ausgangsmaterial zum Wiederaufbau von Gewebe nach einem der Ansprüche 1 und 2, worin die hydrolysierbaren in vivo Bindungen Esterbindungen sind.

4. Ausgangsmaterial zum Wiederaufbau von Gewebe nach Anspruch 3, worin die Vernetzung eine beliebige Bindung von Urethanbindungen, Amidbindungen, Carbamidbindungen, Etherbindungen, Sulfidbindungen, und Schiffscher-Basen-Bindungen sind.

5. Ausgangsmaterial zum Wiederaufbau von Gewebe nach einem der Ansprüche 1 bis 4, worin der sperrige Substituent jeweils aus einer Gruppe ist, die wenigstens einen Benzolring aufweist oder einer Gruppe, die wenigstens ein tertiäres Butyl aufweist.

6. Ausgangsmaterial zum Wiederaufbau von Gewebe nach einem der Ansprüche 1 bis 5, worin die biokompatiblen Gruppen beliebige von Aminosäuren, Oligopeptiden, Oligosacchariden und Zuckerderivaten sind.

7. Ausgangsmaterial zum Wiederaufbau von Gewebe nach einem der Ansprüche 1 bis 6, worin das lineare Molekül Polyethylenglycol ist, die cyclischen Moleküle α-Cyclodextrine sind, die hydrolysierbaren Bindungen Esterbindungen sind, die biokompatiblen Gruppen mit den sperrigen Substituenten Benzyloxycarbonyl-L-phenylalanine sind.

8. Ausgangsmaterial zum Wiederaufbau von Gewebe nach einem der Ansprüche 1 bis 7, worin das Polyrotaxan-Hydrogel eine poröse Struktur aufweist.

9. Ausgangsmaterial zum Wiederaufbau von Gewebe nach einem der Ansprüche 1 bis 8, worin das Polyrotaxan-Hydrogel die Vernetzung zwischen den cyclischen Molekülen aufweist, zwischen den biokompatiblen Gruppen, oder über Vernetzung linearer Moleküle zwischen den cyclischen Molekülen und den biokompatiblen Gruppen, und wobei das Polyrotaxan-Hydrogel die Eigenschaft (a) aufweist:

(a) worin die Zeit ($t_\infty$) für die vollständige Hydrolyse des Polyrotaxan-Hydrogels unabhängig von dessen Wassergehalt ist, jedoch mit jeweils der Abnahme des Polyrotaxangehalts im Polyrotaxan-Hydrogel, dem Anstieg im Molverhältnis der vernetzenden linearen Moleküle zu den cyclischen Molekülen und einer Abnahme des durchschnittlichen Molekulargewichts der vernetzenden linearen Moleküle verlängert wird.

10. Ausgangsmaterial zum Wiederaufbau von Gewebe nach einem der Ansprüche 1 bis 8, worin das Polyrotaxan-Hydrogel die Vernetzung zwischen den cyclischen Molekülen aufweist, zwischen den biokompatiblen Gruppen, oder über Vernetzung linearer Moleküle zwischen den cyclischen Molekülen und den biokompatiblen Gruppen, und wobei das Polyrotaxan-Hydrogel die Eigenschaft (b) aufweist:

(b) wobei das Zerfallsmuster, das als Graph mit $t/ t_\infty$ als Abszisse und $M_t/M_0$ als Ordinate dargestellt ist, wobei $t_\infty$ die Zeit bis zur vollständigen Hydrolyse des Polyrotaxan-Hydrogels ist, t die Zeit des Einweichens des Polyrotaxan-Hydrogels in Wasser ist, $M_0$ das Anfangsgewicht des Polyrotaxan-Hydrogels im durch Wasser gequollenen Zustand ist, und $M_t$ das Gewicht des Polyrotaxan-Hydrogels zum Zeitpunkt t ist, unabhängig vom Polyrotaxangehalt im Polyrotaxan-Hydrogel ist, aber vom Molekulargewicht der vernetzenden linearen Moleküle abhängt.

11. Ausgangsmaterial zum Wiederaufbau von Gewebe nach einem der Ansprüche 9 und 10, worin die cyclischen Moleküle α-Cyclodextrine sind, das lineare Molekül Polyethylenglykol ist, die hydrolysierbaren in vivo Bindungen Esterbindungen sind, die vernetzenden linearen Moleküle Polyethylenglycol-bis-amine sind, und worin das Polyrotaxan-Hydrogel eine Netzwerkstruktur aufweist in der OH-Gruppen der α-Cyclodextrine, die in benachbarten Polyrotaxanmolekülen enthalten sind, über Urethanbindungen von $NH_2$-Gruppen an beiden Enden der Polyethylenglycol-bis-amine vernetzt werden.

12. Ausgangsmaterial zum Wiederaufbau von Gewebe nach einem der Ansprüche 1 bis 11, für die Verwendung zur Kultur von Mesenchymzellen, Leberzellen und Neuronen.

13. Implantierbares Material, dass Zellen umfasst, die in einem Ausgangsmaterial zum Wiederaufbau von Gewebe nach einem der Ansprüche 1 bis 12 kultiviert oder eingebettet sind.

14. Verfahren zum Herstellen eines implantierbaren Materials, wobei das Verfahren ein implantierbares Material bereitstellt, indem Zellen, die in einem Ausgangsmaterial zum Wiederaufbau von Gewebe nach einem der Ansprüche 1 bis 12 kultiviert oder eingebettet sind, hergestellt werden.

**Revendications**

1. Matériau de base pour la reconstruction de tissu qui est principalement composé d'un hydrogel de polyrotaxane, où le polyrotaxane a des groupes biocompatibles, chacun d'entre eux ayant un substituant volumineux, qui sont introduits via des liaisons hydrolysables in vivo à l'une ou l'autre extrémité d'une molécule linéaire avec de multiples molécules cycliques enfilées sur celle-ci, dans lequel l'hydrogel de polyrotaxane a une structure de réseau formée par la réticulation entre les molécules cycliques, entre les groupes biocompatibles, ou entre les molécules cycliques et les groupes biocompatibles dans des molécules de polyrotaxane adjacentes.

2. Matériau de base pour la reconstruction de tissu selon la revendication 1, dans lequel la molécule linéaire est l'un quelconque du polyéthylèneglycol, du polypropylèneglycol, de copolymères de polyéthylèneglycol et de polypropylèneglycol, et du polyméthyl vinyl éther, et la molécule cyclique est l'une quelconque des $\alpha$-, $\beta$-, et $\gamma$-cyclodextrines.

3. Matériau de base pour la reconstruction de tissu selon l'une ou l'autre des revendications 1 et 2, dans lequel les liaisons hydrolysables in vivo sont des liaisons ester.

4. Matériau de base pour la reconstruction de tissu selon la revendication 3, dans lequel la réticulation est des liaisons quelconques parmi des liaisons uréthane, des liaisons amide, des liaisons carbamide, des liaisons éther, des liaisons sulfure, et des liaisons des bases de Schiff.

5. Matériau de base pour la reconstruction de tissu selon l'une quelconque des revendications 1 à 4, dans lequel le substituant volumineux est l'un ou l'autre d'un groupe ayant au moins un cycle benzène et d'un groupe ayant au moins un groupe butyle tertiaire.

6. Matériau de base pour la reconstruction de tissu selon l'une quelconque des revendications 1 à 5, dans lequel les groupes biocompatibles sont l'un quelconque d'acides aminés, d'oligopeptides, d'oligosaccharides, de dérivés du sucre.

7. Matériau de base pour la reconstruction de tissu selon l'une quelconque des revendications 1 à 6, dans lequel la molécule linéaire est le polyéthylèneglycol, les molécules cycliques sont les $\alpha$-cyclodextrines, les liaisons hydrolysables in vivo sont des liaisons ester, les groupes biocompatibles ayant les substituants volumineux sont des benzyloxycarbonyl-L-phénylalanines.

8. Matériau de base pour la reconstruction de tissu selon l'une quelconque des revendications 1 à 7, dans lequel l'hydrogel de polyrotaxane a une structure poreuse.

9. Matériau de base pour la reconstruction de tissu selon l'une quelconque des revendications 1 à 8, dans lequel l'hydrogel de polyrotaxane a la réticulation entre les molécules cycliques, entre les groupes biocompatibles, ou entre les molécules cycliques et les groupes biocompatibles via des molécules linéaires de réticulation, et dans lequel l'hydrogel de polyrotaxane a les caractéristiques (a) :

   (a) dans lequel une durée pour l'hydrolyse complète de l'hydrogel de polyrotaxane ($t_\infty$) dépend d'une teneur en eau dans l'hydrogel de polyrotaxane, mais est allongée avec l'une quelconque d'une diminution de la teneur en polyrotaxane dans l'hydrogel de polyrotaxane, d'une augmentation du rapport molaire des molécules linéaires de réticulation sur les molécules cycliques, et d'une diminution de la masse moléculaire moyenne des molécules linéaires de réticulation.

10. Matériau de base pour la reconstruction de tissu selon l'une quelconque des revendications 1 à 8, dans lequel l'hydrogel de polyrotaxane a la réticulation entre les molécules cycliques, entre les groupes biocompatibles, ou entre les molécules cycliques et les groupes biocompatibles via des molécules linéaires de réticulation, et dans lequel l'hydrogel de polyrotaxane a les caractéristiques (b) :

    (b) dans lequel un motif de décomposition présenté sous la forme d'un graphique avec $t/t_\infty$ en tant que l'axe des abscisses et $M_t/M_0$ en tant que l'axe des ordonnées, où $t_\infty$ est une durée pour l'hydrolyse complète de l'hydrogel de polyrotaxane, t est une durée d'immersion de l'hydrogel de polyrotaxane dans l'eau, $M_0$ est un

poids initial de l'hydrogel de polyrotaxane dans un état gonflé par l'eau, et $M_t$ est un poids de l'hydrogel de polyrotaxane au moment t, est indépendant d'une teneur en polyrotaxane dans l'hydrogel de polyrotaxane, mais dépendant d'une masse moléculaire des molécules linéaires de réticulation.

**11.** Matériau de base pour la reconstruction de tissu selon l'une quelconque des revendications 9 et 10, dans lequel les molécules cycliques sont des α-cyclodextrines, la molécule linéaire est le polyéthylèneglycol, les liaisons hydrolysables in vivo sont des liaisons ester, les molécules linéaires de réticulation sont des polyéthylèneglycol bis-amines, et dans lequel l'hydrogel de polyrotaxane a une structure de réseau, dans lequel les groupes OH des α-cyclodextrines comprises dans les molécules de polyrotaxane adjacentes sont réticulés via des liaisons uréthane de groupes $NH_2$ sur les deux extrémités des polyéthylèneglycol bis-amines.

**12.** Matériau de base pour la reconstruction de tissu selon l'une quelconque des revendications 1 à 11, destiné à être utilisé pour la culture de cellules quelconques choisies parmi des cellules mésenchymateuses, des cellules hépatiques, et des neurones.

**13.** Matériau implantable, comprenant des cellules cultivées dans ou incorporées sur un matériau de base pour la reconstruction de tissu selon l'une quelconque des revendications 1 à 12.

**14.** Procédé de préparation d'un matériau implantable, ledit procédé fournissant un matériau implantable par la culture de cellules dans ou l'incorporation de cellules sur un matériau de base pour la reconstruction de tissu selon l'une quelconque des revendications 1 à 12.

FIG.1

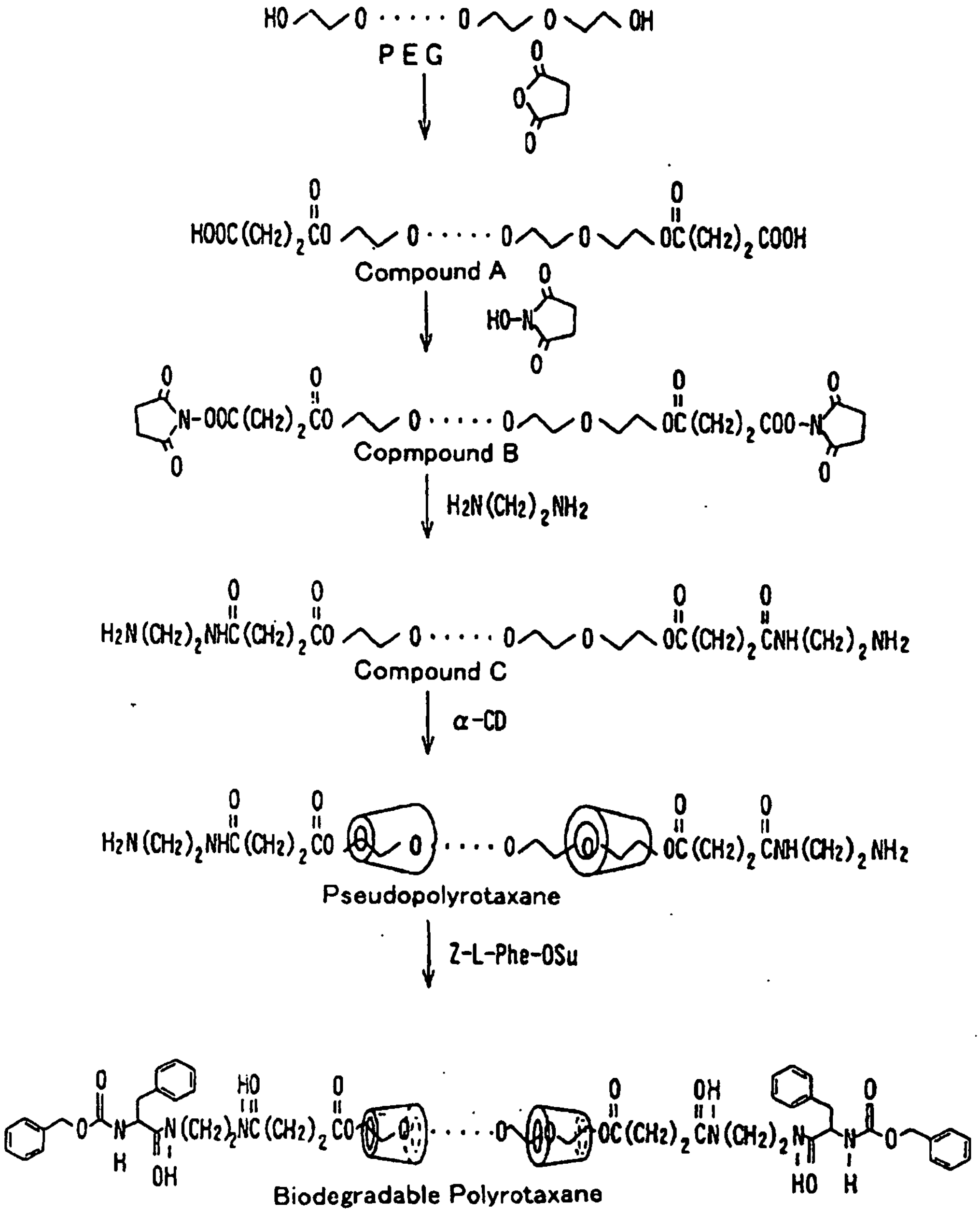
PEG
Compound A
Copmpound B
$H_2N(CH_2)_2NH_2$
Compound C
α-CD
Pseudopolyrotaxane
Z-L-Phe-OSu
Biodegradable Polyrotaxane

FIG.2

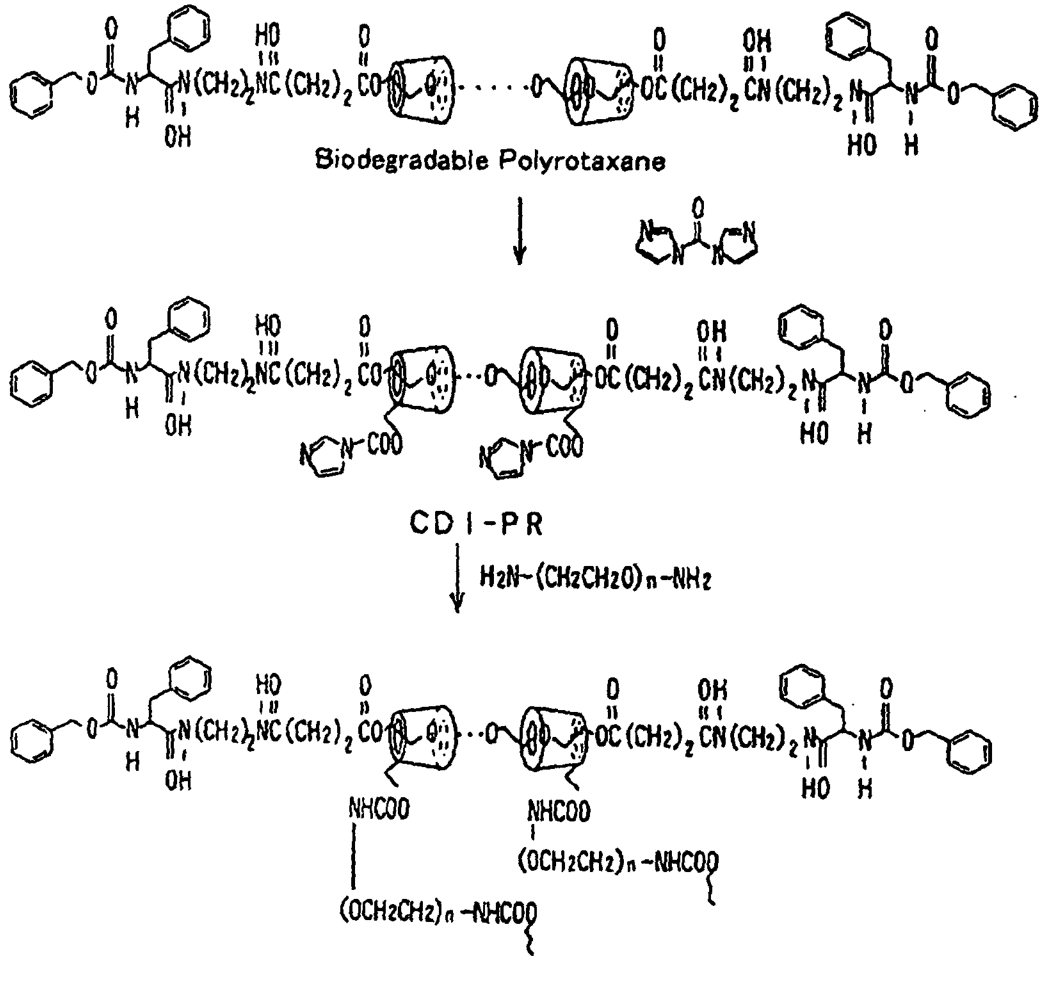


Biodegradable Polyrotaxane

CDI-PR

Polyrotaxane Hydrogel

FIG.3

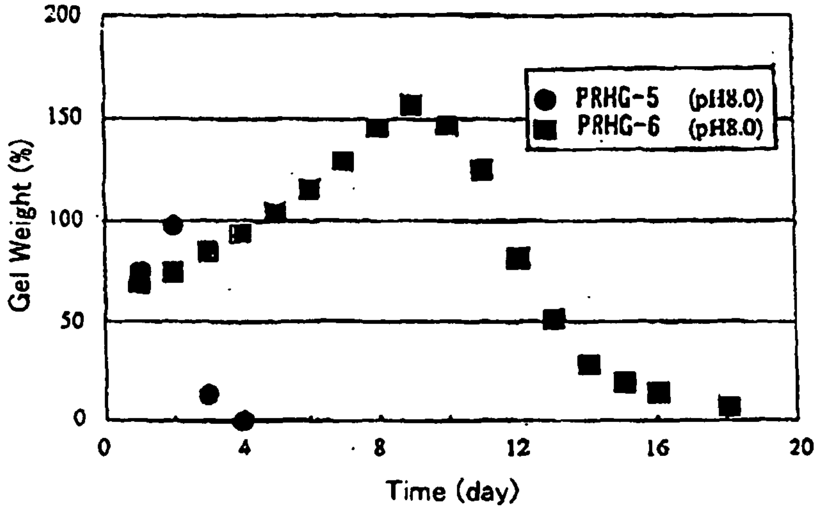
PRHG-5 (pH8.0)
PRHG-6 (pH8.0)
Gel Weight (%)
Time (day)
200
150
100
50
0
0
4
8
12
16
20

FIG.4

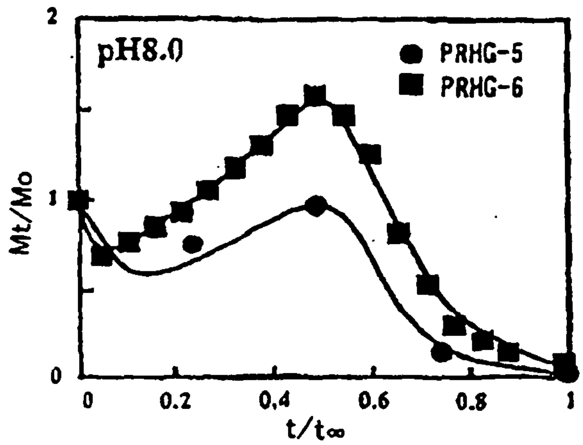
pH8.0
PRHG-5
PRHG-6
Mt/Mo
t/t∞
2
1
0
0
0.2
0.4
0.6
0.8
1

FIG.5

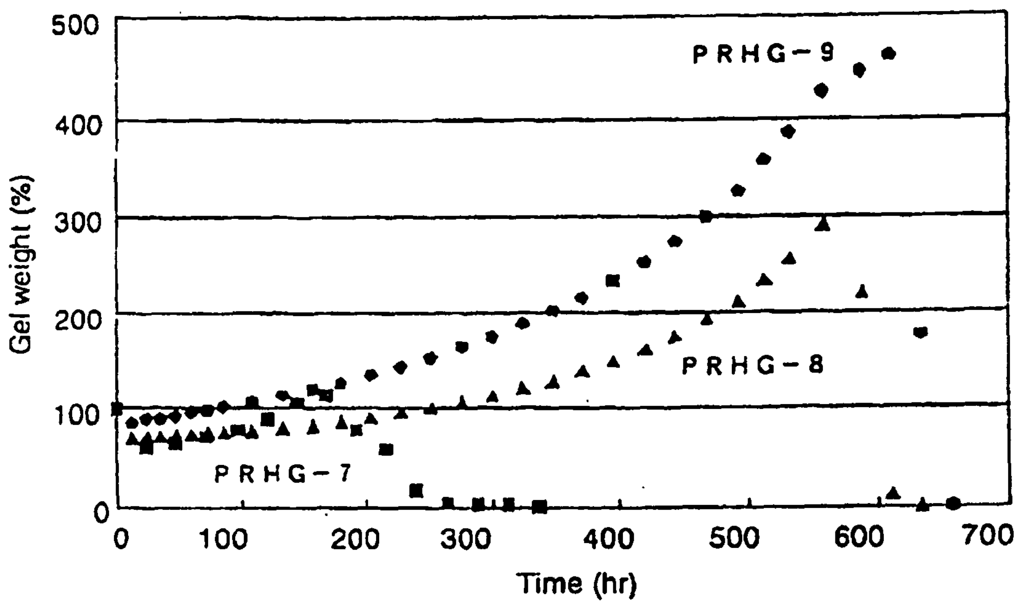

Gel weight (%)
500
400
300
200
100
0
0
100
200
300
400
500
600
700
Time (hr)
PRHG-9
PRHG-8
PRHG-7


FIG.6

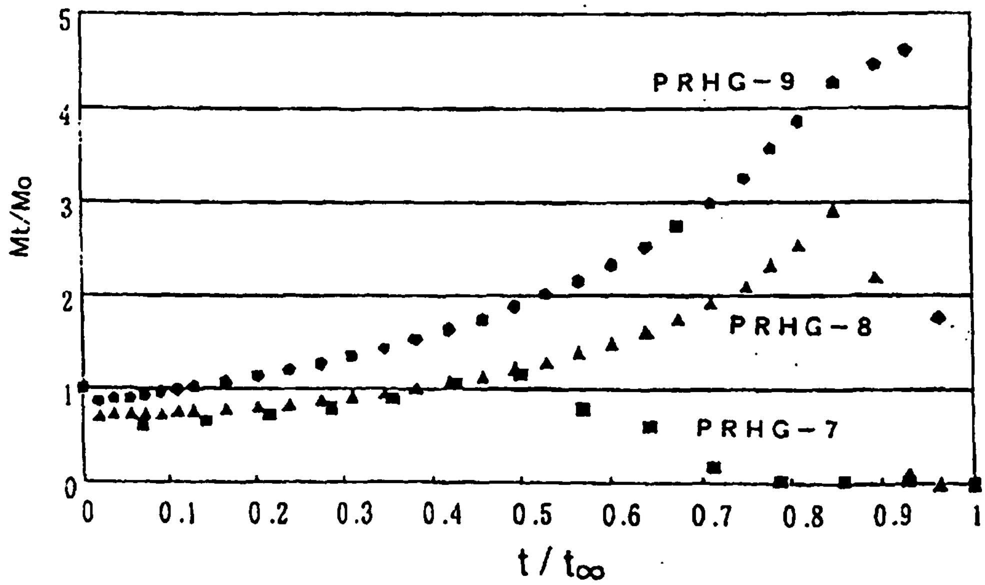

Mt/Mo
5
4
3
2
1
0
0
0.1
0.2
0.3
0.4
0.5
0.6
0.7
0.8
0.9
1
t / t∞
PRHG-9
PRHG-8
PRHG-7

FIG.7

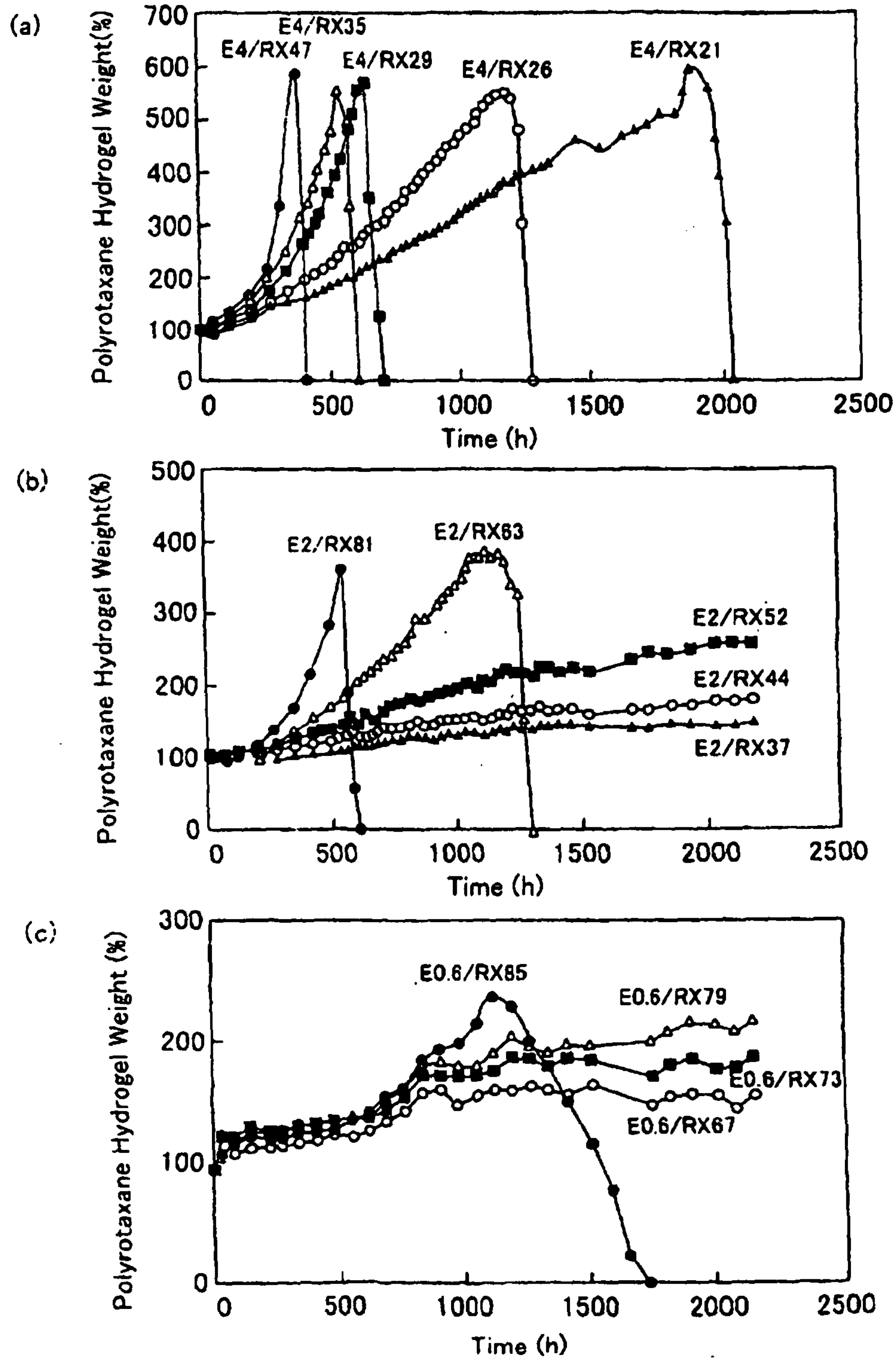
(a)
Polyrotaxane Hydrogel Weight(%)
E4/RX35
E4/RX47
E4/RX29
E4/RX26
E4/RX21
Time (h)
(b)
Polyrotaxane Hydrogel Weight(%)
E2/RX81
E2/RX63
E2/RX52
E2/RX44
E2/RX37
Time (h)
(c)
Polyrotaxane Hydrogel Weight (%)
E0.6/RX85
E0.6/RX79
E0.6/RX73
E0.6/RX67
Time (h)

FIG.8

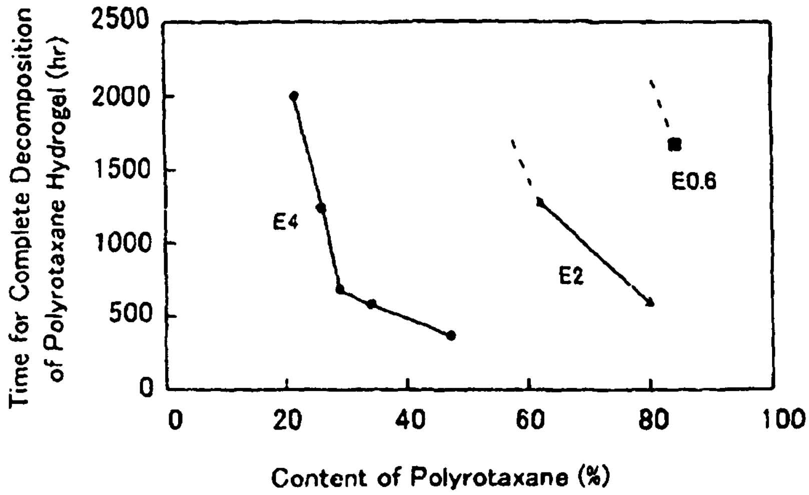
Time for Complete Decomposition of Polyrotaxane Hydrogel (hr)
2500
2000
1500
1000
500
0
0
20
40
60
80
100
Content of Polyrotaxane (%)
E4
E2
E0.6

FIG.9

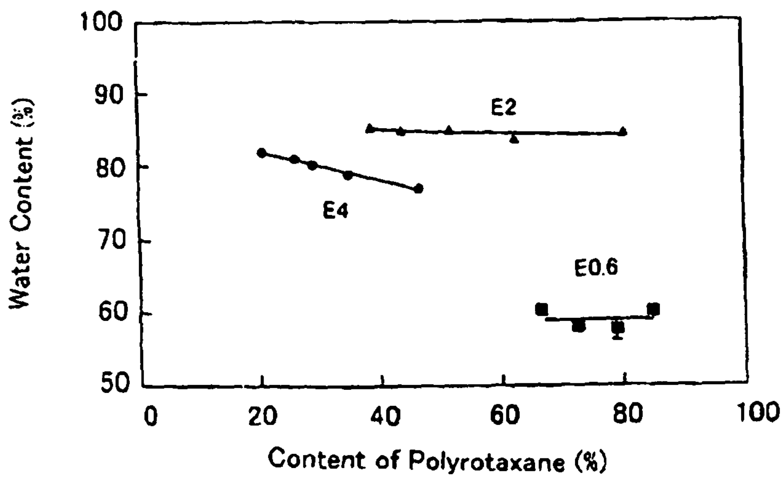
Water Content (%)
100
90
80
70
60
50
0
20
40
60
80
100
Content of Polyrotaxane (%)
E2
E4
E0.6

FIG.10

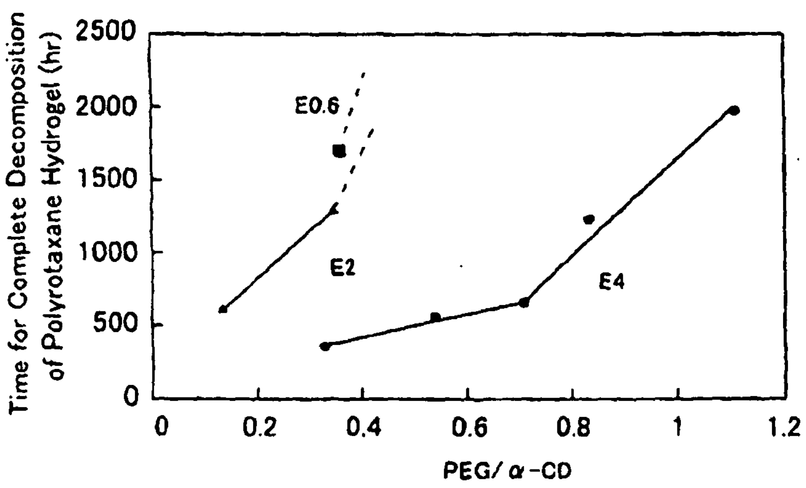
Time for Complete Decomposition of Polyrotaxane Hydrogel (hr)
2500
2000
1500
1000
500
0
0
0.2
0.4
0.6
0.8
1
1.2
PEG/α-CD
E0.6
E2
E4

FIG.11

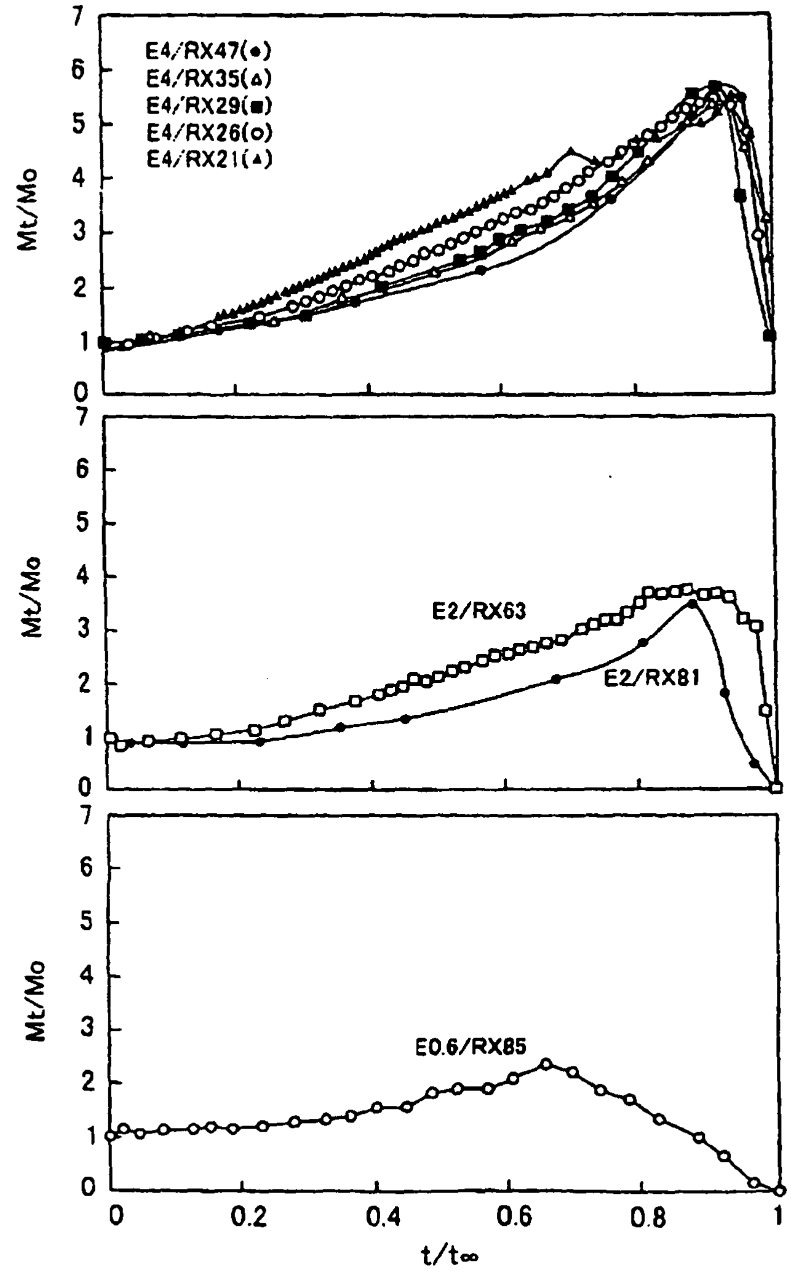
E4/RX47(●)
E4/RX35(△)
E4/RX29(■)
E4/RX26(○)
E4/RX21(▲)
Mt/Mo
E2/RX63
E2/RX81
E0.6/RX85
0
0.2
0.4
0.6
0.8
1
t/t∞

FIG. 12

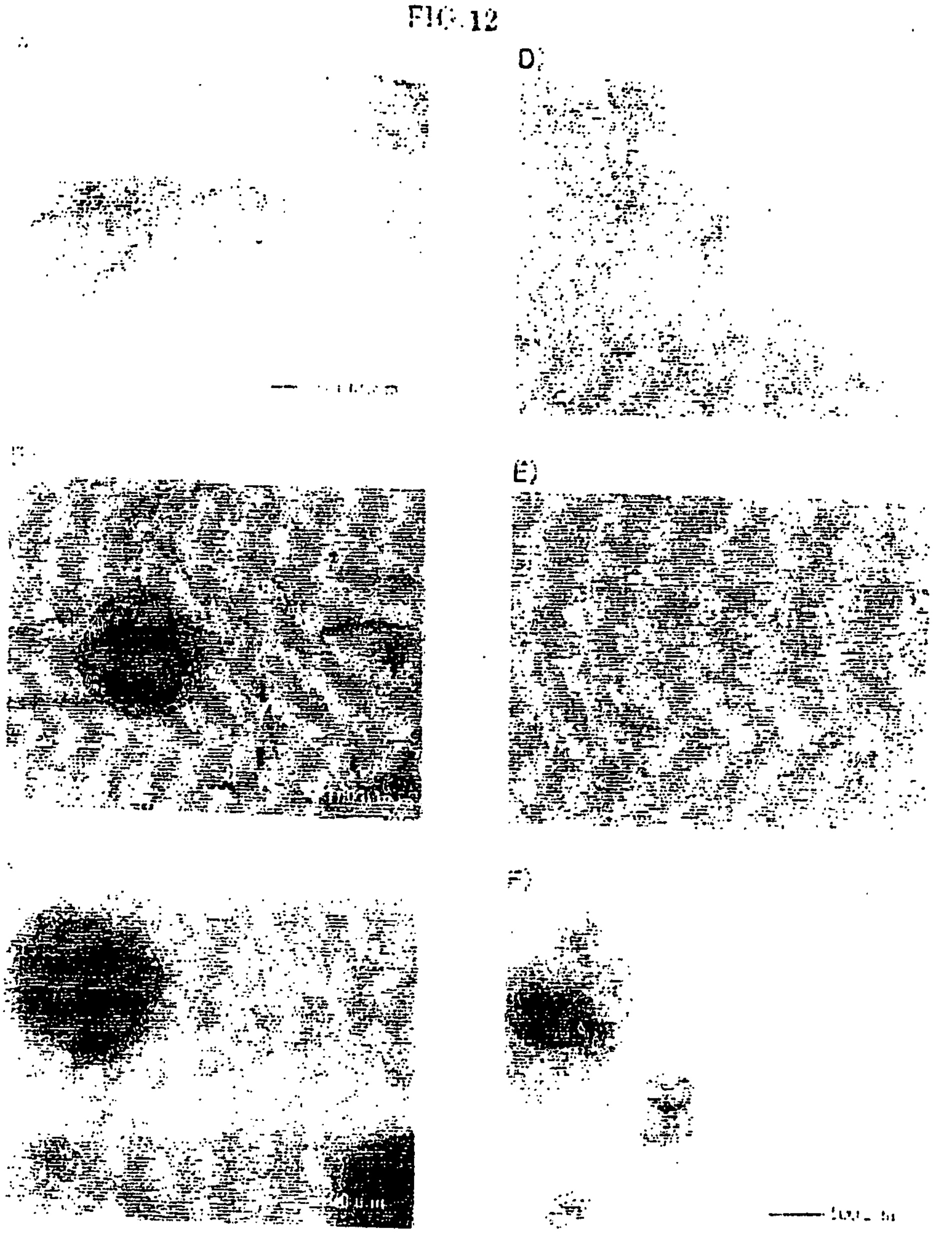


PRHG-1
A) Culture Day 14
B) Culture Day 28
C) Culture Day 42

PRHG-3
D) Culture Day 14
E) Culture Day 28
F) Culture Day 42